(19) Europäisches Patentamt
European Patent Office
Office européen des brevets

(11) Veröffentlichungsnummer: **0 507 004 B1**

(12) **EUROPÄISCHE PATENTSCHRIFT**

(45) Veröffentlichungstag der Patentschrift: **19.04.95**

(51) Int. Cl.[6]: **A61K 7/06**, A61K 7/48, C11D 1/12, C07H 15/08

(21) Anmeldenummer: **91122042.4**

(22) Anmeldetag: **21.12.91**

# (54) Verwendung von Alkylglycosidsulfosuccinaten zur Herstellung von kosmetischen Präparaten und Reinigungsmitteln.

(30) Priorität: **04.04.91 DE 4110852**

(43) Veröffentlichungstag der Anmeldung:
**07.10.92 Patentblatt 92/41**

(45) Bekanntmachung des Hinweises auf die Patenterteilung:
**19.04.95 Patentblatt 95/16**

(84) Benannte Vertragsstaaten:
**AT BE CH DE DK ES FR GB GR IT LI LU NL SE**

(56) Entgegenhaltungen:
**EP-A- 0 358 216**
**US-A- 4 687 843**

(73) Patentinhaber: **Witco Surfactants GmbH**
**Industriegebiet West**
**D-36396 Steinau an der Strasse (DE)**

(72) Erfinder: **Hintz, Mathias**
**Am Schlossborn 17**
**W-6490 Schlüchtern-Hohenzell (DE)**
Erfinder: **Köhle, Hans-Jürgen, Dr. Dipl.-Ing.**
**Lotichiusstrasse 133**
**W-6490 Schlütern (DE)**
Erfinder: **Möller, Christl**
**Bergwinkelstrasse 2**
**W-6497 Steinau an der Strasse (DE)**
Erfinder: **Salomon, Thomas**
**Kalkofenstrasse 5**
**W-6483 Bad-Soden-Salmünster-Wahlert (DE)**
Erfinder: **Weigand, Joachim, Dr. Dipl.-Chem.**
**Haupstrasse 71**
**W-6463 Freigericht 1 (DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung ist die Verwendung von alkoxylierten, Estergruppen enthaltenden Sulfosuccinaten auf Basis von Alkylglycosiden zur Herstellung von milden kosmetischen Präparaten und wässrigen Zubereitungen, welche diese Verbindungen enthalten.

Kosmetische Präparate, insbesondere solche, die in den Bereich der Haar- und Körperreinigung fallen wie Duschbäder, Schaumbäder, Haarshampoos, Babyshampoos, Babybäder, flüssige Seifen, enthalten als Reinigungskomponenten hauptsächlich Aniontenside wie Carboxylate, Alkylsulfate und Alkylethersulfate.

Diese Zubereitungen sollen die Hautoberfläche reinigen, vorzugsweise nur den auf ihr anhaftenden Film, welcher aus Körperausscheidungen wie Schweiß und Fetten, Hautschuppen oder abgelagertem Schmutz aus der Umgebung bestehen kann. Die Reinigungsmittel sollen keinesfalls die Haut auslaugen, sie reizen oder ihre natürliche Funktion beeinträchtigen.

Da die Präparate aber bei ihrer häufigen - in den letzten Jahren fast täglichen - Anwendung zu Irritationen der Haut führen können, werden zur Verbesserung der Haut- und Augenschleimhautverträglichkeit häufig sogenannte milde Tenside mitverwendet, wie beispielsweise Betaine, Proteinderivate, Ampholyte, Alkylethercarboxylate und Sulfosuccinate.

Insbesondere für Babypflegemittel und Babyshampoos sowie Präparate für empfindliche Haut wird besonderer Wert auf extrem niedrige Gehalte an die Haut und Augenschleimhaut reizenden Substanzen gelegt. Die konventionell wegen ihrer ausgezeichneten Reinigungs- und Schaumbildungseigenschaften verwendeten anionischen Tenside können durch die bekannten milden Co-Tenside in ihrer Irritationswirkung zwar weitgehend gemildert werden, in der Praxis werden aber noch Verbesserungen insbesondere in Richtung auf Augenschleimhautverträglichkeit gefordert.

In hohen Konzentrationen oder allein sind die milden Tenside zwar weitgehend irritationsfrei, zeigen dann jedoch keine praxisgerechten Schäumungs- und Reinigungseigenschaften und unbefriedigende Viskositäten.

Ein weiteres Kriterium für die Brauchbarkeit der oberflächenaktiven Substanzen ist ihre Toxizität. Die Toxizität liegt in den Tensiden selbst oder ihren durch Wechselwirkung mit den Bestandteilen der Rezeptur entstehenden Produkten.

Aufgrund des gewachsenen Umweltbewußtseins werden weiterhin Produkte verlangt, welche auf natürlichen nachwachsenden Rohstoffen basieren, keinerlei Toxizität aufweisen und in den kommunalen Kläranlagen schnell und vollständig ohne toxische Zwischenprodukte abgebaut werden können.

Aus der GB-PS 2 011 462 sind polyoxyalkylierte Mono- und Dicarboxylate von $\alpha$-Methylglucosiden bekannt, wobei als einziges Tensid das GLUCAMATE$^R$ SSE-20 der Fa. Amerchol - eine Mischung des Mono- und Distearats von $\alpha$-Methylglucosid - offenbart ist. Es wird angewendet in hautfreundlichen Präparaten zum Abschminken der Augen.

In der DE-OS 33 36 760 werden Mischungen aus mindestens einem oligomeren Alkylglucosidether mit einem Alkylrest mit 8 - 10 C-Atomen, oxyethyliertem Methylglucosiddioleat, Alkyl- oder Hydroxyalkylpolyglycosiden mit einem Alkylrest mit 11 - 18 C-Atomen und zwischen 3 - 25 Glycosideinheiten und mindestens einem nichtionischen Polymer aus der Gruppe der Alkylcellulosen, Polyhydroxyalkylcellulosen, Poly-$\beta$-alaninen, Polyvinylpyrrolidon als milde Haar- und Körperreinigungsmittel beschrieben.

In der Praxis zeigen hochmolekulare Verbindungen manchmal anwendungstechnische Probleme: Sie sind oft schwer in Wasser aufzulösen und erfordern relativ viel Aufwand bei der Einarbeitung, bisweilen ist es schwierig, sofort die gewünschte Viskosität zu erzielen oder die Formulierungen neigen zum "Fädenziehen" und erzeugen ein klebriges Gefühl auf der Haut. Einige dieser Nachteile können herstellungsbedingt sein und könnten möglicherweise durch entsprechende apparative Maßnahmen und sorgfältige Überwachung der Prozeßführung überwunden werden.

Aufgabe der vorliegenden Erfindung ist es, diese Nachteile des Standes der Technik zu überwinden und milde, hautfreundliche kosmetische Präparate, insbesondere solche, die in den Bereich der Haar- und Körperreinigung fallen, zur Verfügung zu stellen.

Diese Aufgabe wird gelöst durch die erfindungsgemäß mitverwendeten alkoxylierten, Estergruppen enthaltenden Sulfosuccinate auf Basis von Alkylglycosiden. Gegenstand der Erfindung ist daher die Verwendung von Alkylglycosidsulfosuccinaten der allgemeinen Formel (1)

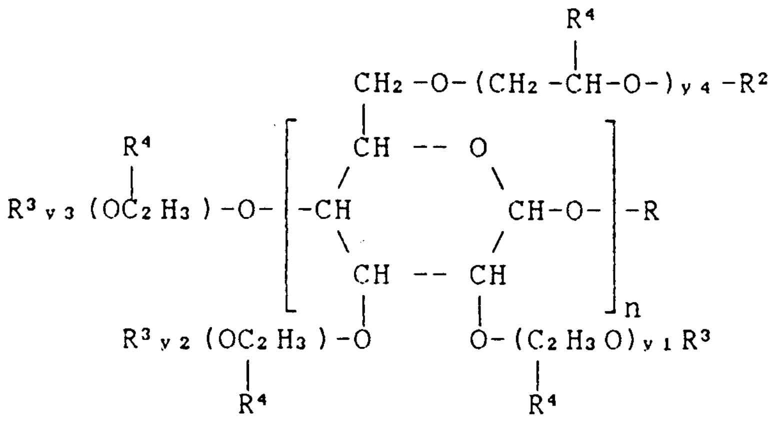

R = linearer gesättigter oder ungesättigter Kohlen wasserstoffrest mit 1 - 18, vorzugsweise 1 - 10, insbesondere 1 - 4 C-Atomen oder

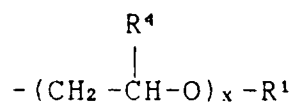

mit $R^1$ = H oder R und x = 1 - 10, vorzugsweise 1 - 5

$R^2$ = ein linearer, gesättigter oder ungesättiger Acylrest mit 8 - 22 C-Atomen, vorzugsweise 12 - 18 C-Atomen

$R^3$ = unabhängig voneinander H und mindestens einmal der Rest $[CO\text{-}CH(SO_3\text{-})\text{-}CH_2\text{-}COO\text{-}]2K^+$ sein kann und

$R^4$ = unabhängig voneinander H oder $CH_3$ sein kann

n = eine Zahl zwischen 1 bis 6, vorzugsweise 1 bis 3 und

$y^{1-4}$ = O - 5 sein kann, wobei $n(y^1 + y^2 + y^4) + y^3 = n(1\text{-}20) + y^3$ vorzugsweise $n(1\text{-}10) + y^3$, insbesondere $n(1\text{-}3) + y^3$ ist und

$K^+$ = beliebiges Kation Alkalimetall (Na), Erdalkali, Ammonium oder H ist

zur Herstellung kosmetischer Präparate und Reinigungsmittel.

Ein weiterer Gegenstand der Erfindung sind wässrige Zubereitungen enthaltend

A) 5 - 20 Gew.-% mindestens eine der Verbindungen gemäß der allgemeinen Formel (1)

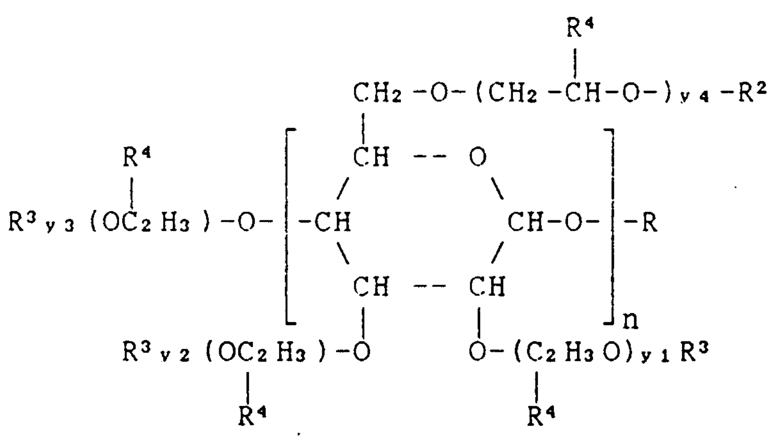

R = linearer gesättigter oder ungesättigter Kohlenwasserstoffrest mit 1 - 18, vorzugsweise 1 - 10, insbesondere 1 - 4 C-Atomen oder

$-(CH_2-CH_2-O)_x-R^1$ mit $R^1$ = H oder R und x = 1 - 10, vorzugsweise 1 - 5

$R^2$ = ein linearer, gesättigter oder ungesättiger Acylrest mit 8 - 22 C-Atomen, vorzugsweise 12 - 18 C-Atomen

$R^3$ = unabhängig voneinander H und mindestens einmal der Rest $[CO-CH(SO_3-)-CH_2-COO-]2K^+$ sein kann und

$R^4$ = unabhängig voneinander H oder $-CH_3$ sein kann

n = eine Zahl zwischen 1 bis 6, vorzugsweise 1 bis 3 und

$y^{1-4}$ = O - 5 sein kann, wobei $n(y^1 + y^2 + y^4) + y^3 = n(1\text{-}20) + y^3$ vorzugsweise $n\ (1\text{-}10) + y^3$ ist

$K^+$ = beliebiges Kation Alkalimetall (Na), Erdalkali, Ammonium oder H

B) 1 - 5 Gew.-% mindestens eines Verdickungsmittels

C) 0 - 10 Gew.-% eines Elektrolytsalzes

D) 0 - 10 Gew.-% üblicher Hilfs- und Zusatzstoffe

E) 55 - 94 Gew.-% Wasser.

Weitere Gegenstände der Erfindung sind durch die Ansprüche gekennzeichnet.

Ein Ausgangsstoff für die Herstellung der erfindungsgemäß mitverwendeten Verbindungen der allgemeinen Formel (1) sind handelsübliche Monomere und Oligomere der Glucose, z. B. Glucosesirup der Fa. Cerestar.

Der Polymerisationsgrad der Glucose variiert je nach Verfahren und Hersteller und liegt im Bereich von 1 - 10 (n = 1 - 10), so daß die erfindungsgemäßen Verbindungen ebenfalls Mischungen dieser verschiedenen Polymerisationsgrade sind. Obwohl der maximale Polymerisationsgrad bei n = ca. 10 liegt, hat der größte Teil einen Polymerisationsgrad (n) von kleiner als 5. Der Durchschnittswert von n kann daher auch eine Dezimalzahl sein, das heißt, zwischen zwei ganzen Zahlen liegen.

Die erfindungsgemäß beanspruchten Polymerisationsgrade von n = 1 bis 6 beinhalten daher auch die anteilsmäßig geringen Mengen mit Werten >6. Erfindungsgemäß bevorzugt werden Polymerisationsgrade mit n ≤3 und insbesondere 1 - 2 mit Schwerpunkt um 1 - 1,5.

Diese werden nach allgemein üblichen Verfahren verethert bzw. umgeethert (vgl. US-PS 4 704 453, EP-A-0 301 298).

Die Gruppe R des Glycosides ist vorzugsweise eine Alkyl- oder Alkenylgruppe mit 1 - 10, insbesondere 1 - 4 C-Atomen und kann gegebenenfalls durch höhere Gruppen mit bis zu 18 C-Atomen ersetzt werden. R kann weiterhin eine Polyether-Gruppe der Struktur $-(-CH_2-CH_2-O-)_x-R^1$ sein, worin $R^1$ = H oder R ist und x Werte zwischen 1 - 10, vorzugsweise 1 - 5 annehmen kann.

In einem zweiten Schritt erfolgt die Umsetzung von einem Mol Fettsäure bzw. Fettsäuremethyl- oder Fettsäureethylester pro Glycosideinheit. Anstelle der Fettsäuren oder deren Methyl- bzw. Ethylester können auch die natürlichen Fette und Oele, das heißt deren Glycerinester, eingesetzt werden.

Als Fettsäuren werden die einbasischen Säuren mit 8 - 22 C-Atomen - bevorzugt die natürlich vorkommenden - verwendet wie Laurinsäure, Myristinsäure, Palmitinsäure, Stearinsäure, Oelsäure, Linolsäure, Linolensäure, Ricinolsäure, Kokosfettsäure oder deren Mischungen (vergl. J. Am. Oil Chemists' Soc. 38, 517 - 520 (1961) und EP-A 0 190 779).

Die Glycosidester werden nach an sich bekannten Verfahren alkoxyliert, vorzugsweise ethoxyliert oder propoxyliert (J. Am. Oil Chemists Soc, 38, 517 - 520 (1961), US-PS 3 640 998). Die Ethoxylierung bzw. Propoxylierung kann auch vor der Einführung der Esterfunktion erfolgen. Die Mengen an Ethylenoxid und/oder Propylenoxid werden so gewählt, daß der Anlagerungsgrad zwischen 1 - 20 pro Glycosidmolekül beträgt, wobei erfindungsgemäße Werte bevorzugt >5, insbesondere bei 8 - 25 liegen. Die Reaktion zum Sulfosuccinat erfolgt mit Maleinsäureanhydrid und anschließender Sulfierung mit Natriumsulfit nach an sich bekannten Verfahren (vgl. DE-OS 27 00 072).

Dabei wird pro Mol umzusetzende Hydroxylgruppe mit einem Mol Maleinsäureanhydrid bei 60 - 80 °C bis zur vollständigen Reaktion des Anhydrids gerührt. Der Maleinsäurehalbester wird anschließend in eine wässrige Natriumsulfit-Lösung gegeben (1 Equivalent Sulfit pro Equivalent Halbester) und bei 60 - 80 °C sulfiert, bis das Sulfit vollständig abreagiert ist. Dann wird das wässrige Produkt pH-neutral eingestellt.

Während handelsübliche Sulfosuccinate nur in Konzentrationen bis ca. 40 Gew.-% in Wasser gelöst werden können, haben die erfindungsgemäßen Verbindungen überraschenderweise den Vorteil, daß sie sich in Konzentration von 50 Gew.-% und höher als klare, niedrigviskose Lösungen herstellen lassen.

Diese Verbindungen, welche allein oder in Mischung mitverwendet werden, können in Konzentrationen von 5 bis 20, vorzugsweise von 6 bis 12 Gew.-%, bezogen auf die Gesamtmischung, einsetzt werden.

Die Anzahl der anionischen Gruppen im Molekül wird beeinflußt durch die Art, wie die Glycosidmoleküle miteinander verknüpft sind. Wenn die Glycosidringe in 1,4- oder 1,3-Position miteinander verknüpft sind, ist

es aus sterischen Gründen einfacher, alle Hydroxylgruppen zu substituieren, als wenn die Verknüpfung in 1,6- oder 1,2-Stellung erfolgt ist.

Obwohl theoretisch 2n+1 anionische Gruppen in den Glycosidester eingebracht werden können, werden erfindungsgemäß 2n bis n Gruppen bevorzugt.

Die erfindungsgemäßen Mischungen liegen im allgemeinen als wässrige Mittel oder als wässrige alkoholische Lösungen, Cremes, Emulsionen, Gele vor und können zur Anpassung an den vorgesehenen Anwendungszweck noch die jeweils üblichen Hilfs- und Zusatzstoffe enthalten, welche außer in wässrigen Reinigungsmitteln, wie milden Geschirrspülmitteln in der manuellen Anwendung, insbesondere für die Herstellung kosmetischer Präparate im Bereich der Haar- und Körperreinigung, das heißt für Duschbäder, Schaumbäder, Shampoos, flüssige Seifen, Babypflegemittel und Babywaschmittel üblich sind, mitverwendet werden.

Dies sind Schaumstabilisierungsmittel wie Polysaccaride, Carboxyvinylpolymere, Polyvinylpyrrolidone, Fettsäurepartialglyceridpolyglykolether,Eiweiß-Fettsäure-Kondensate;
Perlglanz- und Trübungsmittel wie Fettalkohole, Ethylenglykoldistearate, Monoester mehrwertiger Alkohole mit höheren Fettsäuren, Polystyrolemulsionen;
Komplexbildner oder Sequestierungsmittel wie Salze der Ethylendiamintetraessigsäure;
Konservierungsmittel wie Sorbinsäure, Citronensäure, Ascorbinsäure, Chlorhexidin, 8-Hydroxichinolin und seine Salze, Benzalkoniumchlorid, Dimethylalkylbenzylammoniumchlorid und geringe Mengen an Duftstoffen, Farbstoffen, hautkosmetischen Wirkstoffen, Pflanzenextrakten, Puffersubstanzen.

Als Verdickungsmittel gemäß B) können erfindungsgemäß handelsübliche Fettsäurepartialglyceridpolyglykolether wie REWODERM$^R$ LI 420, REWODERM$^R$ LI 48 (Warenzeichen der Fa. REWO Chemische Werke GmbH, Steinau an der Straße) in Mengen von 1 bis 10, vorzugsweise von 2 bis 4 Gew.-%, bezogen auf die Gesamtmischung, mitverwendet werden.

Als Elektrolytsalze eignen sich alle Alkali-, Erdalkali- und Ammoniumsalze, welche bei 20 °C in Mengen von mindestens 1 Gew.-% löslich sind. Bevorzugt werden Natriumchlorid, Magnesiumchlorid und Ammoniumchlorid, welche in Mengen bis zu ca. 10 Gew.-%, vorzugsweise 1 - 5 Gew.-%, bezogen auf die Gesamtformulierung, mitverwendet werden können.

Der in den nachfolgenden Beispielen angegebene Gehalt an waschaktiver Substanz (B-WAS) wird durch die übliche Zweiphasentitration mit Benzalkoniumchlorid gegen Methylenblau als Indikator titriert (vgl. S. R. Epton, Nature (London), 160, 1967, S. 795).

Die Verseifungszahl ist ein Maß für die in Fetten und technischen Fettsäuren enthaltenen freien und gebundenen Säuren. Sie gibt die Anzahl Milligramm Kaliumhydroxid an, die notwendig ist, um 1 Gramm Substanz oder technische Fettsäuren zu verseifen (mg KOH/g). Die Werte werden bestimmt nach den Einheitsmethoden der Deutschen Gesellschaft für Fettchemie (DGF): DGF C-V3.

Die Hydroxylzahl dient zur Ermittlung des Gehalts an Hydroxylgruppen und gibt die Anzahl Milligramm Kaliumhydroxid an, die notwendig ist, um die von 1 Gramm Substanz bei der Acetylierung verbrauchte Essigsäure zu neutralisieren (mg KOH/g). Die Werte werden bestimmt nach der DGF-Einheitsmethode DGF C-V17a.

Der Feststoffgehalt wird bestimmt durch Trocknen bei 105 °C bis zur Gewichtskonstanz.

Herstellung der erfindungsgemäßen Verbindungen

Beispiel 1

Herstellung des Butylglucosidkokosfettsäureesters

Zu 826 g (3,5 mol) n-Butylglucosid werden 8 g $K_2CO_3$ gegeben und die Reaktionsmischung auf 120 °C aufgeheizt. Bei 50 mbar werden 750 g Kokosmethylester über 2 h zur Reaktionsmischung getropft und anschließend 4 h bei 120 °C und 30 mbar nachreagiert. Das entstehende Methanol wird dabei sofort abdestilliert. Es entsteht eine gelbe hochviskose Masse mit folgenden Analysenzahlen:

| | |
|---|---|
| Verseifungszahl | 110 mg KOH/g |
| Hydroxylzahl | 430 mg KOH/g |

Beispiel 2

Herstellung des Polyethylenoxidethers

463 g (1,05 mol) Butylglucosidester aus Beispiel 1 werden mit 2,3 g KOH versetzt und bei 120 °C im Autoklaven portionsweise mit 464 g (10,5 Mol) Ethylenoxid versetzt, so daß der Druck maximal 5 bar beträgt. Es entsteht eine hochviskose gelbe Masse mit folgenden Analysenzahlen:

| | |
|---|---|
| Verseifungszahl | 54 mg KOH/g |
| Hydroxylzahl: | 223 mg KOH/g |

Beispiel 3

Herstellung des Sulfosuccinats

258 g (0,3 mol) Butylglucosidesterethoxylat aus Beispiel 2 werden mit 57,6 g (0,6 mol) Maleinsäureanhydrid bei 70 °C 2 h gerührt. Anschließend wird der Maleinsäurehalbester in eine 60 °C warme Lösung aus 350 g Wasser mit 74,1 g (0,6 mol) $Na_2SO_3$ gegeben und bei dieser Temperatur gerührt, bis der $SO_2$-Gehalt der Lösung <0,01 % ist. Es entsteht eine klare Lösung mit 48,3 % Feststoffgehalt und 43,5 % Benzavlon-waschaktive Substanz (B-WAS).

Die folgenden Beispiele wurden in Anlehnung an die Beispiele 1 - 3 durchgeführt.

Beispiel 4

258 g (0,3 mol) Butylglucosidesterethoxylat aus Beispiel 2 werden wie in Beispiel 3 beschrieben mit 29 g (0,3 mol) Maleinsäureanhydrid umgesetzt und anschließend in einer Lösung aus 300 g Wasser mit 37 g (0,3 mol) $Na_2SO_3$ sulfiert. Die Lösung hat einen Feststoffgehalt von 49 % und eine B-WAS von 41 %.

Beispiel 5

258 g (0,3 mol) Butylglucosidesterethoxylat aus Beispiel 2 werden wie in Beispiel 3 beschrieben mit 87 g (0,9 mol) Maleinsäureanhydrid umgesetzt und anschließend mit einer Lösung aus 400 g Wasser mit 111 g (0,9 mol) $Na_2SO_3$ sulfiert. Die Lösung hat einen Feststoffgehalt von 47 % und eine B-WAS von 43 %.

Beispiel 6

200 g (0,3 mol) Butylglucosidesterethoxylat (wie Beispiel 2, jedoch $\Sigma Y = 5$) werden mit 59 g (0,6 mol) Maleinsäureanhydrid bei 80 °C umgesetzt. Wie in Beispiel 1 beschrieben wird anschließend der Halbester in einer Lösung aus 330 g Wasser und 77 g (0,61 mol) $Na_2SO_3$ sulfiert. Das Sulfosuccinat hat 49 % Feststoff und eine B-WAS von 41 %.

Beispiel 7

200 g (0,3 mol) des Butylglucosidesterethoxylats wie in Beispiel 6 beschrieben werden mit 88,5 g (0,9 mol) Maleinsäureanhydrid bei 80 °C umgesetzt. Analog Beispiel 3 erfolgt die Sulfierung in 400 g Wasser und 116 g $Na_2SO_3$. Das Sulfosuccinat hat 50 % Feststoff und eine B-WAS von 44 %.

Beispiel 8

200 g (0,3 mol) Butylglucosidesterethoxylat wie in Beispiel 6 werden mit 29,5 g (0,3 mol) Maleinsäureanhydrid bei 75 °C umgesetzt. Analog Beispiel 3 erfolgt die Sulfierung in 270 g Wasser mit 38 g (0,3 mol) $Na_2SO_3$. Das Sulfosuccinat hat 48,2 % Feststoff bei 28,1 % B-WAS.

Beispiel 9

220 g (0,5 mol) Butylglucosidester aus Beispiel 1 werden analog Beispiel 3 mit 49 g (0,5 mol) Maleinsäureanhydrid umgesetzt und anschließend in 500 g Wasser mit 63 g (0,5 mol) $Na_2SO_3$ sulfiert. Das Sulfosuccinat hat 38,3 % Feststoff und eine B-WAS von 23,2 %.

Beispiel 10

238 g (1 mol) Butylglucosid werden mit 0,5 g Kaliumhydroxid versetzt und bei 140 °C im Autoklaven portionsweise mit 440 g (10 mol) Ethylenoxid versetzt, so daß der maximale Druck 5 bar nicht übersteigt. Es entsteht ein viskoses, gelbes Produkt mit einer Hydroxylzahl von 360.

Beispiel 11

678 g (1 mol) des Produkts aus Beispiel 10 werden mit 3 g $K_2CO_3$ versetzt und unter einer $N_2$-Atmosphäre auf 120 °C aufgeheizt. Unter Vakuum von 100 mbar werden über ca. 2 h 210 g (0,95 mol) Kokosmethylester zur Reaktionslösung getropft und anschließend noch 4 h bei 120 °C nachreagiert. Das entstehende Methanol wird kontinuierlich aus dem System entfernt. Es entsteht eine hellgelbe, viskose Masse mit folgenden Analysenzahlen:

| | |
|---|---|
| Verseifungszahl | 60 |
| Hydroxylzahl | 240 |

Beispiel 12

890 g (1 Mol) Butylglucosid-Ethoxylat-Ester aus Beispiel 11 werden wie im Beispiel 3 beschrieben mit 98 g (1 mol) Maleinsäureanhydrid umgesetzt und anschließend mit einer Lösung aus 1100 g Wasser und 126 g $Na_2SO_3$ sulfiert. Es entsteht eine helle Lösung mit einem Feststoffgehalt von 49 % und einer B-WAS von 31 %.

Tabelle 1

| Beispiel | R | $R^2$ | $R^3$ | $R^4$ | ΣY | $Y^4$ | n |
|---|---|---|---|---|---|---|---|
| 1* | n-Butyl | Cocoacyl | 3 x H | H | 0 | 0 | ca.1,3 |
| 2* | n-Butyl | Cocoacyl | 3 x H | H | 10 | 0 | ca.1,3 |
| 3 | n-Butyl | Cocoacyl | 1 x H, 2 x Sulfosuccinat | H | 10 | 0 | ca.1,3 |
| 4 | n-Butyl | Cocoacyl | 2 x H, 1 x Sulfosuccinat | H | 10 | 0 | ca.1,3 |
| 5 | n-Butyl | Cocoacyl | 3 x Sulfosuccinat | H | 10 | 0 | ca.1,3 |
| 6 | n-Butyl | Cocoacyl | 1 x H, 2 x Sulfosuccinat | H | 5 | 0 | ca.1,3 |
| 7 | n-Butyl | Cocoacyl | 3 x Sulfosuccinat | H | 5 | 0 | ca.1,3 |
| 8 | n-Butyl | Cocoacyl | 2 x H, 1 x Sulfosuccinat | H | 5 | 0 | ca.1,3 |
| 9 | n-Butyl | Cocoacyl | 2 x H, 1 x Sulfosuccinat | H | 0 | 0 | ca.1,3 |
| 10* | n-Butyl | H | 3 x H | H | 10 | ca.1-3 | ca.1,3 |
| 11* | n-Butyl | Cocoacyl | 3 x H | H | 10 | ca.1-3 | ca.1,3 |
| 12 | n-Butyl | Cocoacyl | 2 x H, 1 x Sulfusuccinat | H | 10 | ca.1-3 | ca.1,3 |

Cocoacyl = Mischung aus natürlichen $C_{12-16}$-Fettsäuren

* = Vorstufeprodukte

Sulfosuccinat = $-CO-CH(SO_3^-)-CH_2-COO^-$ $2Na^+$ bzw.

$-CO-CH_2-CH-(SO_3^-)-COO^-$ $2 Na^+$

Alle Rezepturen werden in Gewichtsprozent auf Feststoff berechnet angegeben.

Erklärung der eingesetzten Tenside nach Cosmetic, Toiletry and Fragrance Association (CTFA)-Normen:

1) Disodium Laureth Sulfosuccinate
2) Sodium Laureth Sulfate
3) Cocoamidopropyl Betaine
4) PEG-80 Glyceryl Tallowate
5) Glyceryl Stearate
6) PEG-200 Glyceryl Tallowate mod.

7) Disodiumcocoamphodiacetate

Grundrezeptur Haarshampoo

| | | |
|---|---|---|
| Beispiel 4 oder 12 | 2 - 6 | Gew.-Teile |
| REWOPOL® NL 3-28 | 6 - 15 | " |
| REWODERM® LI S 75[6] | 1 - 3 | " |
| demin. Wasser | ad 100 | |

Grundrezeptur Babyshampoo bzw. Shampoo für empfindliche Haut

| | | |
|---|---|---|
| Beispiel 4 oder 12 | 3 - 8 | Gew.-Teile |
| REWOTERIC® AM 2 C NM[7] | 2 - 10 | " |
| REWOTERIC® AM B 13 H[3] | 4 - 8 | " |
| REWODERM® LI 48-50[4] | 1 - 4 | " |
| demin. Wasser | ad 100 | |

Grundrezeptur Creme

| | | |
|---|---|---|
| Beispiel 4 oder 12 | 1 - 5 | Gew.-Teile |
| Glycerinmonodistearat | 2 - 10 | " |
| Cetylalkohol | 1 - 4 | " |
| Paraffinoel 3,5 °E | 4 - 12 | " |
| Glycerin | 1 - 5 | " |
| demin. Wasser | ad 100 | |
| Konsiervierungsmittel | n. B. | |

Grundrezeptur Duschbad

| | | |
|---|---|---|
| Beispiel 4 oder 12 | 2 - 10 | Gew.-Teile |
| REWOTERIC® AM B 13 | 2 - 8 | " |
| REWOPOL® NL 3-28 | 5 - 15 | " |
| REWODERM® LI 48-50 | 1 - 4 | " |
| demin. Wasser | ad 100 | |

Grundrezeptur Spülmittel

| | | |
|---|---|---|
| Beispiel 12 | 1 - 5 | Gew.-Teile |
| REWOPOL® NL 3-28 | 1 - 30 | " |
| REWOTERIC® AM CAS | 1 - 5 | " |
| REWOPOL® LA 6 | 1 - 10 | " |
| demin. Wasser | ad 100 | |

Anwendungstechnische Überprüfung

**Duschbad**

Die entwickelten Glycosidderivate sind in der Testrezeptur von einem Testpanel von 20 Personen (weiblich und männlich) bewertet worden bezüglich

- Anschäumvermögen, Schaumstruktur
- Hautgefühl der nassen Haut
- Hautgefühl der abgetrockneten Haut

Hautverträglichkeit (Zeinwerte) vergleiche:
Z. Götte "Hautverträglichkeit von Tensiden, gemessen am Lösevermögen für Zein" Chem. Phys. Appl. Surface Active Subst. Proc. Int. Congr. 4 (1964) 83 - 90.

Bewertungsschema: < 200 mgN/100 ml = nicht irritierend
200 - 400 mgN/100 ml = leicht irritierend

> 400 mgN/100 ml = irritierend

**Tellerwaschtest**

1. Prinzip

Es wird die Anzahl künstlich angeschmutzter Teller bestimmt, die sich bis zur Erschöpfung der Flotte in einer Spülmittellösung reinigen lassen.

2. Herstellung des Testschmutzes

In eine 50 °C warme Schmelze aus 48,0 Gew.-Teilen Schweineschmalz werden nacheinander 2,0 Gew.-Teile technischer Oelsäure, 49,9 Gew.-Teile Weizenmehl und 0,1 Gew.-Teil des Farbstoffes Sudanrot eingerührt und homogenisiert.

3. Beschichtung der Teller

Die Teller werden zunächst gewaschen, mit klarem Wasser gespült und mit Isopropanol nachgespült. Auf jeden getrockneten Teller werden sodann auf der Innenfläche 2 g des 40 °C warmen Testschmutzes mit einem Pinsel gleichmäßig verteilt und anschließend 24 h bei 20 °C und 65 % relativer Luftfeuchtigkeit gelagert.

4. Herstellung der Spülflotten

Spülflotte A:

In einer Schüssel werden 8 l von 50 °C warmem Leitungswasser mit 10 °deutscher Härte (°dH) und einem Gehalt der zu überprüfenden Testsubstanz von 0,02 Gew.-% (bezogen auf Feststoff) vorgelegt und 2 min mit einem Flügelrührer mit 300 rpm gerührt, so daß ausreichend Schaum entsteht.

Spülflotte B:

In einer zweiten Schüssel werden 10 l kaltes Leitungswasser (20 °C 10 °dH) vorgelegt, welches kontinuierlich durch zufließendes Frischwasser (5 l/min) erneuert wird.

5. Spülvorgang

Die angeschmutzten Teller werden nacheinander während jeweils 30 sec unter der Oberfläche der Spülflotte A mit der Spülbürste abgewaschen. Anschließend werden sie durch einfaches Eintauchen in Flotte B nachgespült. Es wird solange gespült, bis Flotte A erschöpft ist. Dies erkennt man am Verschwinden des Schaumes, dem visuell erkennbaren Verbleiben von Schmutzteilen auf den gespülten Tellern oder dem Anschwimmen schwarzer bzw. roter Fetteilchen auf der Oberfläche der Flotte A.

6. Auswertung

Es wird durch subjektive Beurteilung von mindestens 3 Personen die Anzahl der einwandfrei sauber gespülten Teller bestimmt. Es werden angegeben:

| | |
|---|---|
| Anzahl der Teller | wie angegeben |
| Konzentration der Spülflotte in g/l | 0,02 % Feststoffgehalt |
| Wasserhärte in mmol $Ca^{++}/1$ | 10 °dH |
| Spültemperatur in °C | 50 |

Duschbadformulierung

| Rezeptur | 1 | 2 | 3 |
|---|---|---|---|
| Beispiel 4 | 5 | - | - |
| Beispiel 12 | - | 5 | - |
| REWOTERIC® AM B 13[3)] | 3 | 3 | 3 |
| REWOPOL® NL 3-28[2)] | 11 | 11 | 16 |
| REWODERM® LI 48-50[4)] | 2 | 2 | 2 |
| demin. Wasser | ad 100 | ad 100 | ad 100 |
| pH-Wert mit Citronensäure eingest. | 6,5 | 6,5 | 6,5 |
| Anschäumvermögen[A] | gute Schaumentwicklung | gute Schaumentwicklung | gute Schaumentwicklung |
| Schaumstruktur[A] | cremig-weich | cremig-weich | grobblasig |
| Hautgefühl nasse Haut[A)] | cremig-weich | cremig-weich | weich |
| Hautgefühl trockene Haut[A)] | glatt-weich | glatt-weich | leicht trocken |

A) = Die Bewertung erfolgte nach einem abgestuften Beurteilungssystem, wobei die oben angeführte Beurteilung den arithmetischen Mittelwert wiedergibt

Hautreinigungspräparat, Reduzierung Zeinwerte

| | 1 | 2 | 3 | 4 |
|---|---|---|---|---|
| Beispiel 4 | 4,5 | | - | - |
| Beispiel 12 | | 4,5 | - | - |
| REWOPOL$^R$ SB FA 30[1)] | - | - | 4,5 | - |
| REWOPOL$^R$ NL 3-28[2)] | 10,5 | 10,5 | 10,5 | 15 |
| demin. Wasser | 85 | 85 | 85 | 85 |
| pH-Wert mit Citronensäure eingestellt | 6,5 | 6,5 | 6,5 | 6,5 |
| Bewertung der Schaumqualität | cremig-sahnig feinblasig | cremig-sahnig feinblasig | feinblasig | grobblasig |
| Hautgefühl nach dem Waschen auf der trockenen Haut | glatt, weich | glatt, weich | weich | etwas rauh |
| Zein-Werte | 120 mgN/100ml | 122 mgN/100ml | 270 mgN/100ml | 300 mgN/100ml |

Haarshampoo

Die entwickelten Glycosidderivate sind in der Rezeptur von 10 in der Anwendung erfahrenen Testpersonen auf

- Kämmbarkeit
- Volumen des Haares und Sitz der Frisur

bewertet worden:

| Rezeptur | 1 | 2 | 3 |
|---|---|---|---|
| Beispiel 4 | 4,5 | - | - |
| Beispiel 12 | - | 4,5 | - |
| REWOPOL$^R$ NL 3-28[2)] | 10,5 | 10,5 | 15 |
| REWODERM$^R$ LI S 75[6)] | 2 | 2 | 2 |
| demin. Wasser | 83 | 83 | 83 |
| pH-Wert mit Citronensäure eingestellt auf | 6,5 | 6,5 | 6,5 |
| Kämmbarkeit | 5 | 6 | 2 |
| Volumen des Haares Sitz der Frisur | 6 | 5 | 3 |

Bewertung der Kämmbarkeit:

1 - 3 schlecht durchzukämmen; das Haar setzt dem Kämmvorgang erheblichen Widerstand entgegen

4 - 7 das Haar läßt sich relativ gut durchkämmen, Kämmwiderstand verringert, je nach Haartyp ausreichend für ein Conditioning-Shampoo

8 -10 vorbehalten für die nachfolgende Nachbehandlung durch sogenannte Hair-Rinse-Mittel

Bewertung des Haarvolumens und des Sitzes der Frisur

1 - 3 das Haar ist trocken oder strohig; schlechter Sitz der Frisur

4 - 7 das Haar ist weich und füllig bei gleichzeitig gutem Sitz der Frisur

8 -10 das Haar ist weich und glatt, jedoch zu locker, dadurch kein guter Sitz der Frisur.

Die Bewertung erfolgte nach einem abgestuften Punktsystem, wobei die oben aufgeführte Beurteilung den arithmetischen Mittelwert wiedergibt.

Hautcreme

Einsatz von wässrigen Sulfosuccinaten auf Glycosidbasis als Emulgatoren in O/W Emulsionen

| | | |
|---|---|---|
| HLB-Wert | Beispiel 4<br>Beispiel 12 | ca. 16<br>ca. 16 |

Bestimmung nach W. C. Griffin ("Calculation of Surface Active Agents by HLB", Journal soc. Cosm. Chem. 1, 311 (1949)).

| Hautcreme O/W Testrezeptur | 1 | 2 | 3 | 4 |
|---|---|---|---|---|
| Beispiel 4 | 2 | - | - | - |
| Beispiel 12 | - | 2 | - | - |
| Stearylalkohol+3EO | - | - | 2 | - |
| REWOMUL® MG[5] | 6 | 6 | 6 | 6 |
| REWOPOL® SB FA 30[1] | - | - | - | 2 |
| Cetylalkohol | 2 | 2 | 2 | 2 |
| Paraffinoel 3,5°E | 8 | 8 | 8 | 8 |
| Glycerin | 3 | 3 | 3 | 3 |
| Konservierungsmittel | n.B. | n.B. | n.B. | n.B. |
| demin. Wasser | ad 100 | ad 100 | ad 100 | ad 100 |
| Stabilität der Emulsion bei 50 °C, 20 °C und 40 °C | stabil | stabil | stabil | instabil |
| Aussehen | glatte weiße Creme | glatte weiße Creme | glatte weiße Creme | griesig, Emulsion bricht |

Bewertung der Eigenschaften auf der Haut durch 10 Testpersonen; mehr als 80 % beurteilten die stabilen Testrezepturen 1 und 2

- als gut auf der Haut zu verstreichen
- als gut in die Haut einziehend
- die Haut wird glatt und weich, ohne klebrig, fettig zu sein. Die Vergleichsrezeptur Nr. 4 wird nicht gleich bewertet und ist darüber hinaus instabil.

| Babyshampoo bzw. Haar- und Körper-Shampoo für empfindliche Haut | | | |
|---|---|---|---|
| Rezeptur | 1 | 2 | 3 |
| Beispiel 4 | 6 | - | - |
| Beispiel 12 | - | 6 | - |
| REWOTERIC$^R$ AM 2C NM[7)] | 6 | 6 | 6 |
| REWOTERIC$^R$ AM B 13[3)] | 4 | 4 | 4 |
| REWODERM$^R$ LI 48-50[4)] | 2 | 2 | 2 |
| REWOPOL$^R$ NL 3-28[2)] | - | - | 6 |
| demin. Wasser | ad 100 | ad 100 | ad 100 |
| pH-Wert eingestellt mit Citronensäure auf | 6,5 | 6,5 | 6,5 |
| Zein-Werte, mgN/100 ml | <70 | <70 | ca. 150 |
| Hautrauhigkeit nach Padberg[B)] | gering aufrauhend | gering aufrauhend | deutlich aufrauhend |
| Die aufgeführte Rezeptur ist eine "nicht irritierende", die Haut nur "gering aufrauhende" Waschlösung. Die Bewertung "gering aufrauhend" ist die niedrigste, die nach der aufgeführten Methode mit Tensiden erreicht werden kann. | | | |

[B)] Prüfung der Hautverträglichkeit nach Padberg J. Soc. Cosm. Chem. 20, 719 - 728 (1969)
Bewertung der Hautrauhigheit nach Padberg:
Note 1 sehr gering aufrauhend - unterscheidet sich nicht vom Leerfeld
Note 2 gering aufrauhend - % Padberg unter 50
Note 3 deutlich aufrauhend - % Padberg über 50
Note 4 stark aufrauhend - unterscheidet sich nicht signifikant vom Natriumlaurylsulfatfeld (Vergleichssubstanz)

| Testrezeptur: Hautfreundliche Geschirrspülmittel zur manuellen Anwendung | | | | |
|---|---|---|---|---|
| Rezeptur | 1 | 2 | 3 | 4 |
| Beispiel 12 | - | 3 | 1,5 | - |
| REWOPOL$^R$ NL 3-28 [7)] | 18 | 18 | 18 | 18 |
| REWOTERIC$^R$ AM CAS [3)] | 1,5 | - | 1,5 | - |
| REWOPOL$^R$ LA 6 [4)] | 1,5 | - | - | 3 |
| Wasser | ad 100 | ad 100 | ad 100 | ad 100 |
| Tellerwaschtest Anzahl der Teller | 12 | 20 | 18 | 12 |
| Zein-Werte (Hautverträglichkeit) | 220 | 195 | 220 | 250 |
| Schaumhöhe (mm) | 195/185 | 190/180 | 195/190 | 190/180 |
| + 0,5 ml Olivenoel | 190/180 | 190/180 | 195/185 | 190/180 |
| + 1,0 ml Olivenoel | 190/185 | 190/180 | 190/180 | 190/180 |

**Patentansprüche**

1. Verwendung von Alkylglycosidsulfosuccinaten der allgemeinen Formel (1)

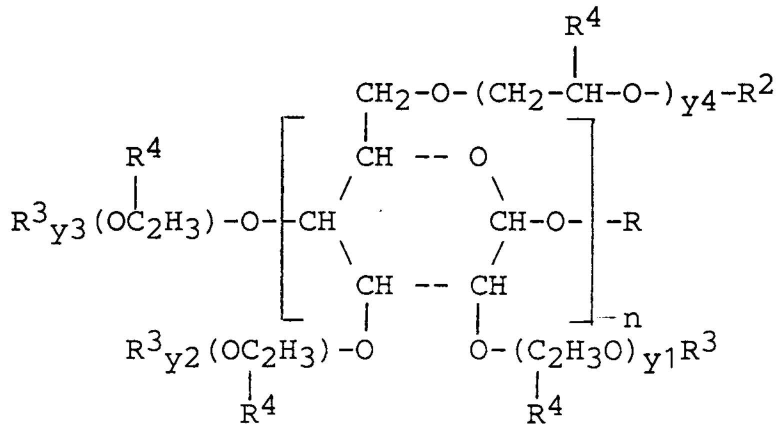

R = linearer gesättigter oder ungesättigter Kohlenwasserstoffrest mit 1 - 18 oder

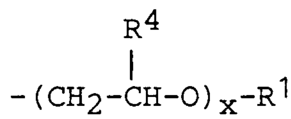

mit $R^1$ = H oder R und x = 1 - 10,

$R^2$ = ein linearer, gesättigter oder ungesättiger Acylrest mit 8 - 22 C-Atomen

$R^3$ = unabhängig voneinander H und mindestens einmal der Rest $[CO\text{-}CH(SO_3\text{-})\text{-}CH_2\text{-}COO\text{-}]2K^+$ sein kann und

$R^4$ unabhängig voneinander H oder $CH_3$ sein kann

n = eine Zahl zwischen 1 bis 6 und

$y^{1-4}$ = O - 5 sein kann, wobei $n(y^1 + y^2 + Y^4) + y^3 = n(1\text{-}20) + y^3$ ist

$K^+$ = beliebiges Kation Alkalimetall (Na), Erdalkali, Ammonium oder H

zur Herstellung kosmetischer Präparate.

2. Verwendung von Alkylglycosidsulfosuccinaten gemäß Anspruch 1, dadurch gekennzeichnet, daß

R = Alkylrest mit 1 - 4 C-Atomen

$R^2$ = Acylrest der Mischung der natürlichen Kokosfettsäuren

$R^3$ = unabhängig voneinander H und mindestens einmal der Rest $[CO\text{-}CH(SO_3\text{-})\text{-}CH_2\text{-}COO\text{-}]2K^+$ sein kann und

$R^4$ = unabhängig voneinander H oder $CH_3$ sein kann

n = 1 - 2

$y^{1-4}$ = 0 - 5, wobei $n(Y^1 + Y^2 + Y^4) + Y^3 = n(1\text{-}3) + Y^3$

$K^+$ = beliebiges Kation Alkalimetall (Na), Erdalkali, Ammonium oder H ist,

zur Herstellung kosmetischer Präparate und Reinigungsmittel.

3. Wässrige Zubereitung enthaltend

A) 5 - 20 Gew.-% mindestens eine der Verbindungen gemäß der allgemeinen Formel (1)

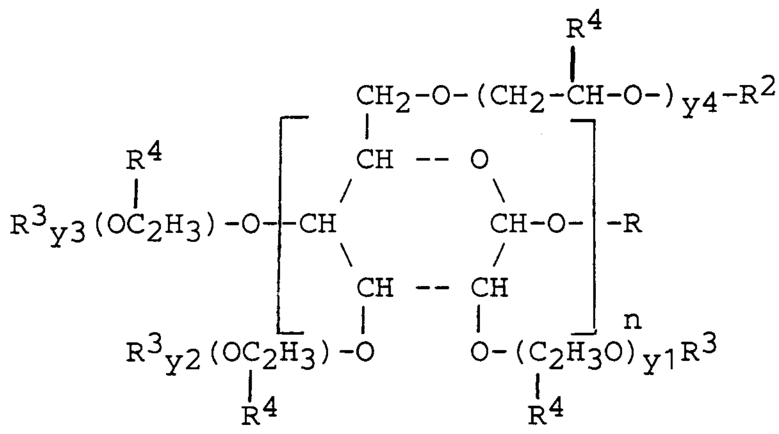

R = linearer gesättigter oder ungesättigter Kohlenwasserstoffrest mit 1 - 18 oder

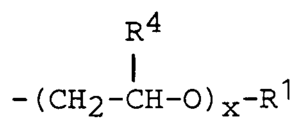

mit $R^1$ = H oder R und x = 1-10,

$R^2$ = ein linearer, gesättigter oder ungesättiger Acylrest mit 8 - 22 C-Atomen

$R^3$ = unabhängig voneinander H und mindestens einmal der Rest $[CO\text{-}CH(SO_3\text{-})\text{-}CH_2\text{-}COO\text{-}]2K^+$ sein kann und

$R^4$ = unabhängig voneinander H oder $CH_3$ sein kann

n = eine Zahl zwischen 1 bis 6 und

$y^{1-4}$ = O - 5 sein kann, wobei $n(y^1 + y^2 + y^4) + y^3 = n(1\text{-}20) + y^3$ ist

$K^+$ = beliebiges Kation Alkalimetall (Na), Erdalkali, Ammonium oder H

B) 1 - 5 Gew.-% mindestens eines Verdickungsmittels

C) 0 - 10 Gew.-% eines Elektrolytsalzes

D) 0 - 10 Gew.-% üblicher Hilfs- und Zusatzstoffe

E) 55 - 94 Gew.-% Wasser.

**4.** Wässrige Haarreinigungsmittel, enthaltend

a) 1 - 10 Gew.-Teile mindestens eine der Verbindungen der allgemeinen Formel (1) und

b) 1 - 20 Gew.-Teile mindestens eines Tensides aus der Gruppe der nichtionischen amphoteren, zwitterionischen, ionischen Tenside und gegebenenfalls

c) 0,1 - 10 Gew.-Teile Verdickungsmittel, Duftstoffe, Konservierungsstoffe, Farbstoffe, Pflanzenextrakte und sonstige Zusatz- und Hilfsstoffe und

d) ad 100 Wasser.

**5.** Wässriges Duschbad, enthaltend

a) 1 - 10 Gew.-Teile der Verbindungen der allgemeinen Formel (1)

b) 1 - 20 Gew.-Teile mindestens eines nichtionischen, amphotren, zwitterionischen, ionischen Tensids und

c) 0,1 - 10 Gew.-Teile Verdickungsmittel, Duftstoffe, Konservierungsmittel, Farbstoffe, Pflanzenextrakte und sonstige Zusatz- und Hilfsstoffe und gegebenenfalls

d) ad 100 Wasser.

**6.** Wässriges Geschirrspülmittel, enthaltend

a) 1 - 10 Gew.-Teile mindestens einer der Verbindungen der allgemeinen Formel (1) und
b) 1 - 30 Gew.-Teile mindestens eines Tensids aus der Gruppe der nichtionischen, amphoteren, zwitterionischen, ionischen Tenside und gegebenenfalls
c) 0,1 - 10 Gew.-Teile Verdickungsmittel, Duftstoffe, Konservierungsmittel, Farbstoffe, Pflanzenextrakte und sonstige Zusatz- und Hilfsstoffe und gegebenenfalls
d) ad 100 Wasser.

**7.** Hautcreme, enthaltend
a) 1 - 10 Gew.-Teile mindestens einer der Verbindungen der allgemeinen Formel (1) und
b) 1 - 10 Gew.-Teile mindestens eines Tensids aus der Gruppe der nichtionischen, amphoteren, zwitterionischen, ionischen Tenside und gegebenenfalls
c) 0,5 - 20 Gew.-Teile pflanzliche oder mineralische Oele, Esteroele
d) 1 - 10 Gew.-Teile Konsistenzgeber
e) 0,5 - 5 Gew.-Teile Duftstoffe, Farbstoffe, Konservierungsmittel
f) ad 100 Wasser.

**8.** Babyshampoo, enthaltend
a) 1 - 10 Gew.-Teile mindestens einer der Verbindungen der allgemeinen Formel (1) und
b) 0,1 - 20 Gew.-Teile mindestens eines Tensids aus der Gruppe der nichtionischen, amphoteren, zwitterionischen, ionischen Tenside und gegebenenfalls
c) 0,1 - 10 Gew.-Teile Verdickungsmittel, Duftstoffe, Konservierungsmittel, Farbstoffe, Pflanzenextrakte und sonstige Zusatz- und Hilfsstoffe und gegebenenfalls
d) ad 100 Wasser.

## Claims

**1.** Use of alkyl glycoside sulphosuccinates of the general formula (1)

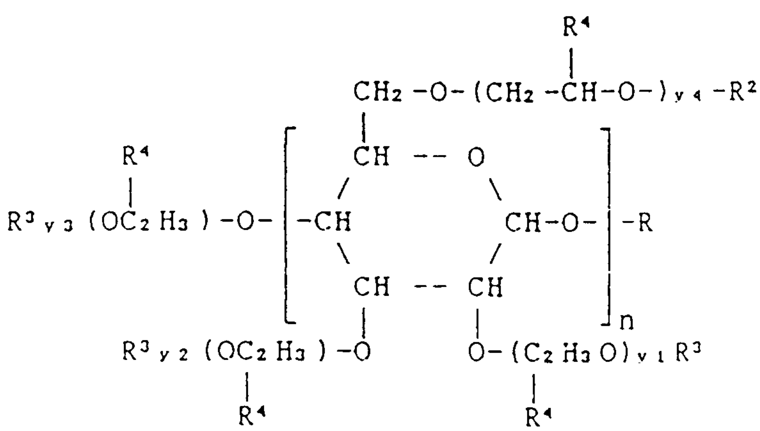

wherein

R is a linear saturated or unsaturated 1-18-hydrocarbon radical or

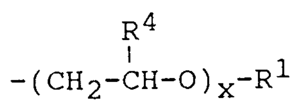

wherein $R^1$ is hydrogen or R and x is from 1 to 10,

$R^2$ is a linear, saturated or unsaturated acyl radical having from 8 to 22 carbon atoms,

each $R^3$, independently of the others, is hydrogen and at least one may be the radical $[CO-CH-(SO_3-)-CH_2-COO-]2K^+$ and

each $R^4$, independently of the others, may be hydrogen or $CH_3$,

n is a number from 1 to 6 and

$y^{1-4}$ may be from 0 to 5, $n(y^1 + y^2 + y^4) + y^3$ being equal to $n(1\text{-}20) + y^3$,
$K^+$ is any desired cation of an alkali metal (Na) or an alkaline earth metal, ammonium or hydrogen,

for the manufacture of cosmetic preparations.

**2.** Use of alkyl glycoside sulphosuccinates according to claim 1, characterised in that

R is an alkyl radical having from 1 to 4 carbon atoms,
$R^2$ is an acyl radical of the mixture of the natural coconut fatty acids,
each $R^3$, independently of the others, is hydrogen and at least one may be the radical [CO-CH-($SO_3$-)-$CH_2$-COO-]2$K^+$ and
each $R^4$, independently of the others, may be hydrogen or $CH_3$,
$\underline{n}$ is 1 or 2,
$\overline{y}^{1-4}$ is from 0 to 5, $n(y^1 + y^2 + y^4) + y^3$ being equal to $n(1\text{-}3) + y^3$,
$K^+$ is any desired cation of an alkali metal (Na) or an alkaline earth metal, ammonium or hydrogen,

for the manufacture of cosmetic preparations and cleaning agents.

**3.** Aqueous preparation containing

A) from 5 to 20 % by weight of at least one of the compounds according to the general formula (1)

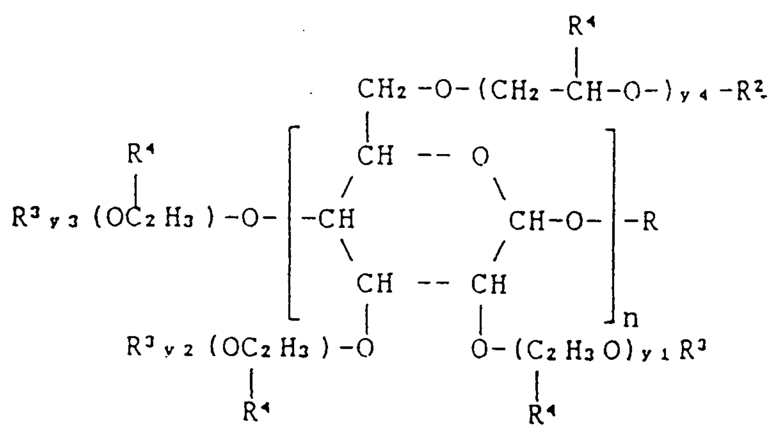

wherein

R is a linear saturated or unsaturated 1-18-hydrocarbon radical or

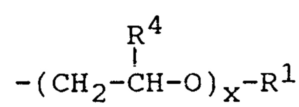

wherein $R^1$ is hydrogen or R and x is from 1 to 10,
$R^2$ is a linear, saturated or unsaturated acyl radical having from 8 to 22 carbon atoms,
each $R^3$, independently of the others, is hydrogen and at least one may be the radical [CO-CH($SO_3$-)-$CH_2$-COO-]2$K^+$ and
each $R^4$, independently of the others, may be hydrogen or $CH_3$,
$\underline{n}$ is a number from 1 to 6 and
$\overline{y}^{1-4}$ may be from 0 to 5, $n(y^1 + y^2 + y^4) + y^3$ being equal to $n(1\text{-}20) + y^3$,
$K^+$ is any desired cation of an alkali metal (Na) or an alkaline earth metal, ammonium or hydrogen,

B) from 1 to 5 % by weight of at least one thickener,
C) from 0 to 10 % by weight of an electrolyte salt,
D) from 0 to 10 % by weight of customary auxiliaries and additives,
E) from 55 to 94 % by weight of water.

**4.** Aqueous hair-cleansing agents, containing

a) from 1 to 10 parts by weight of at least one of the compounds of the general formula (1) and
b) from 1 to 20 parts by weight of at least one surfactant from the group of nonionic, amphoteric, zwitterionic and ionic surfactants, and optionally
c) from 0.1 to 10 parts by weight of thickeners, perfumes, preservatives, colourants, plant extracts and other additives and auxiliaries, and
d) ad 100 water.

**5.** Aqueous shower bath, containing

a) from 1 to 10 parts by weight of the compounds of the general formula (1),
b) from 1 to 20 parts by weight of at least one nonionic, amphoteric, zwitterionic, ionic surfactant and
c) from 0.1 to 10 parts by weight of thickeners, perfumes, preservatives, colourants, plant extracts and other additives and auxiliaries, and optionally
d) ad 100 water.

**6.** Aqueous washing-up agent, containing

a) from 1 to 10 parts by weight of at least one of the compounds of the general formula (1) and
b) from 1 to 30 parts by weight of at least one surfactant from the group of nonionic, amphoteric, zwitterionic and ionic surfactants, and optionally
c) from 0.1 to 10 parts by weight of thickeners, perfumes, preservatives, colourants, plant extracts and other additives and auxiliaries, and optionally
d) ad 100 water.

**7.** Skin cream, containing

a) from 1 to 10 parts by weight of at least one of the compounds of the general formula (1) and
b) from 1 to 10 parts by weight of at least one surfactant from the group of nonionic, amphoteric, zwitterionic and ionic surfactants, and optionally
c) from 0.5 to 20 parts by weight of vegetable or mineral oils, or ester oils,
d) from 1 to 10 parts by weight of an agent imparting consistency,
e) from 0.5 to 5 parts by weight of perfumes, colourants, preservatives,
f) ad 100 water.

**8.** Baby shampoo, containing

a) from 1 to 10 parts by weight of at least one of the compounds of the general formula (1) and
b) from 0.1 to 20 parts by weight of at least one surfactant from the group of nonionic, amphoteric, zwitterionic and ionic surfactants, and optionally
c) from 0.1 to 10 parts by weight of thickeners, perfumes, preservatives, colourants, plant extracts and other additives and auxiliaries, and optionally
d) ad 100 water.

## Revendications

**1.** Utilisation de sulfosuccinates d'alkyl glucosides de formule générale (1)

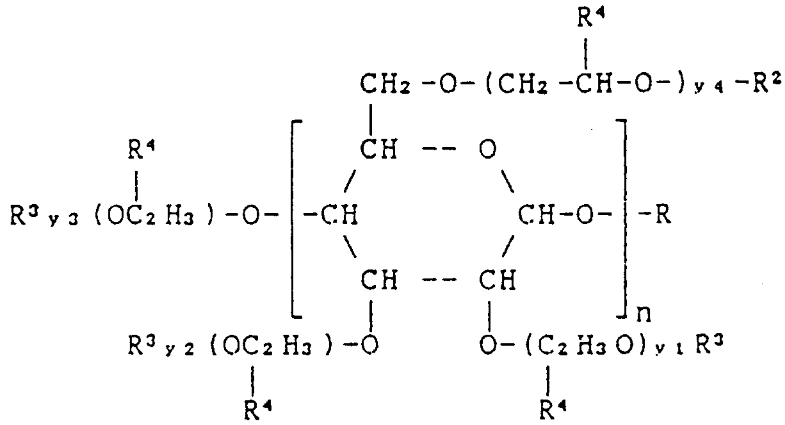

dans laquelle :

R représente un reste d'hydrocarbure linéaire saturé ou insaturé, comportant 1 à 18 atomes de C, ou

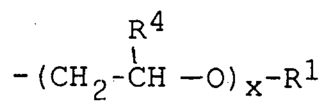

avec $R^1$ = H ou R et x valant 1 à 10,

$R^2$ représente un reste acyle linéaire, saturé ou insaturé, comportant 8 à 22 atomes de carbone,

les $R^3$ peuvent représenter chacun, indépendamment l'un de l'autre, H et au moins une fois le reste $[CO\text{-}CH(SO_3\text{-})\text{-}CH_2\text{-}COO\text{-}]2K^+$ , et

les $R^4$ peuvent représenter chacun, indépendamment l'un de l'autre, H ou $CH_3$,

n est un nombre de valeur comprise entre 1 et 6, et

$y^{1-4}$ peuvent valoir 0 à 5, l'expression $n\ (y^1 + y^2 + y^4) - y^3 = n(1\text{-}20) + y^3$

$K^+$ représente un cation quelconque de métal alcalin (Na), de métal alcalino terreux, un groupe ammonium ou H,

pour la confection de préparations ou compositions cosmétiques.

**2.** Utilisation de sulfosuccinates d'alkylglycosides selon la revendication 1, caractérisée en ce que

R représente un reste alkyle comportant 1 à 4 atomes de carbone,

$R^2$ représente un reste acyle de mélange d'acides gras naturels de noix de coco,

les $R^3$ peuvent représenter chacun, indépendamment l'un de l'autre, H et au moins une fois le reste $[CO\text{-}CH(SO_3\text{-})\text{-}CH_2\text{-}COO\text{-}]2K^+$ , et

les $R^4$ peuvent représenter chacun, indépendamment l'un de l'autre, H ou $CH_3$,

n vaut 1-2

$y^{1-4}$ vaut 0 à 5, l'expression $n\ (y^1 + y^2 + y^4)\ t\ y^3 = n(1\text{-}3) + y^3$

$K^+$ représente un cation quelconque de métal alcalin (Na), de métal alcalino terreux, un groupe ammonium ou H,

pour la confection de préparations ou de compositions cosmétiques et de produits de nettoyage.

**3.** Préparation aqueuse contenant :

A) 5 à 20 % en poids d'au moins l'un des composés répondant à la formule générale (1)

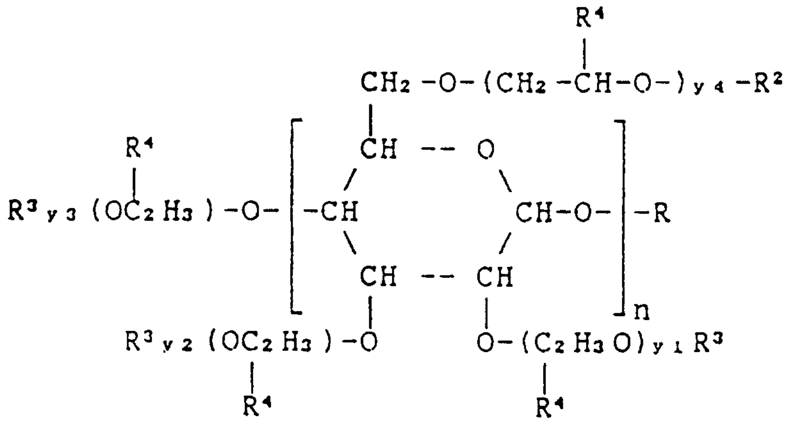

dans laquelle :

- R représente un reste d'hydrocarbure linéaire, saturé ou insaturé, comportant 1 à 18 atomes de carbone ou

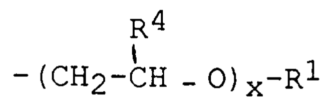

  avec $R^1$ = H ou R et x valant 1 à 10,
- $R^2$ représente un reste acyle linéaire, saturé ou insaturé, comportant 8 à 22 atomes de carbone;
- les $R^3$ peuvent représenter chacun, indépendamment l'un de l'autre, H ou au moins une fois le reste $[CO\text{-}CH(SO_3\text{-})\text{-}CH_2\text{-}COO\text{-}]2K^+$ , et
- les $R^4$ peuvent representer chacun, indépendamment l'un de l'autre, H ou $CH_3$;
- n est un nombre dont la valeur se situe entre 1 et 6, et
- $y^{1-4}$ peut valoir 0 à 5, et $n\ (y^1 + y^2 + y^4) + y^3 = n(1\text{-}20) + y^{3;}$
- $K^+$ représente un cation quelconque de métal alcalin (Na), de métal alcalino terreux, un groupe ammonium ou H;

B) 1 à 5 % en poids d'au moins un épaississant,
C) 0 à 10 % en poids d'un sel électrolyte
D) 0 à 10 % en poids d'adjuvants et additifs usuels,
E) 55 à 94 % en poids d'eau,.

**4.** Produit aqueux pour le nettoyage de la chevelure (produit capillaire), contenant

a) 1 à 10 parties en poids d'au moins l'un des composés répondant à la formule générale (1) et
b) 1 à 20 parties en poids d'au moins un tensio-actif choisi dans le groupe des tensio-actifs non ioniques, amphothères, de type zwiterion, ioniques, et éventuellement
c) 0,1 à 10 parties en poids d'un épaississant, d'un parfum, d'un conservateur, de colorants, d'extraits de plantes et d'autres additifs et adjuvants et,
d) de l'eau pour compléter à 100.

**5.** Produit aqueux pour bain-douche, ce produit contenant :

a) 1 à 10 parties en poids des composés de formule générale (1)
b) 1 à 20 parties en poids d'au moins un tensio-actif non ionique, amphotère, de type zwiterion, ionique, et

c) 0,1 à 10 parties en poids d'un épaississant, de parfums, d'un conservateur, de colorants, d'extraits de plantes et d'autres additifs et adjuvants, et éventuellement ,
d) de l'eau pour compléter à 100.

**6.** Produit aqueux pour rincer la vaisselle, ce produit contenant :
a) 1 à 10 parties en poids d'au moins l'un des composés répondant à la formule générale (1), et
b) 1 à 30 parties en poids d'au moins un tensio-actif choisi dans l'ensemble formé par les tensio-actifs non ioniques, amphotères, de type zwiterion, ioniques et éventuellement
c) 0,1 à 10 parties en poids d'un épaississant, de parfums, d'agents de conservation, de colorant, d'extraits de plantes et d'autres additifs et adjuvants, et éventuellement
d) de l'eau pour compléter à 100.

**7.** Crême pour la peau, cette crême contenant :
a) 1 à 10 parties en poids d'au moins l'un des composés de formule générale (1) et,
b) 1 à 10 parties en poids d'au moins un tensio-actif choisi dans l'ensemble formé par les tensio-actifs non ioniques, amphotères, du type zwiterion, ioniques, et éventuellement
c) 0,5 à 20 Parties en poids d'une huile végétale ou minérale, d'une huile du type ester,
d) 1 à 10 parties en poids d'un agent donnant de la consistance,
e) 0,5 à 5 parties en poids de parfum, de colorant, de conservateur,
f) de l'eau pour compléter à 100

**8.** Shampooing pour bébés, ce shampooing contenant:
a) 1 à 10 parties en poids d'au moins l'un des composés répondant à la formule générale (1) et,
b) 0,1 à 20 parties en poids d'au moins un tensio-actif choisi dans l'ensemble formé par les tensio-actifs non ioniques, amphotères, du type zwiterion, ioniques et éventuellement ,
c) 0,1 à 10 parties en poids d'un épaississant, de parfums, de conservateurs, de colorants, d'extraits de plantes et d'autres additifs, et adjuvants, et éventuellement,
d) de l'eau pour compléter à 100.